# EUROPÄISCHE PATENTSCHRIFT

(11) **EP 0 496 299 B1**
(45) Veröffentlichungstag und Bekanntmachung des Hinweises auf die Patenterteilung: **17.04.1996**
(21) Anmeldenummer: 92100765.4
(22) Anmeldetag: 17.01.1992
(51) Int. Cl.: A61K 31/02, A61K 9/00, A61K 31/08, A61K 31/13

(54) **Verdünnungsmittel für zur Netzhautentfaltung eingesetzte Perfluorcarbone (Entfaltungs-PFCL)**
Diluent for perfluorocarbones used in retina deployment
Diluant pour perfluorocarbures utilisés à déployer la rétine

(30) Priorität: 24.01.1991 DE 4101976
(43) Veröffentlichungstag der Anmeldung: 29.07.1992
(73) Patentinhaber: Chiron Adatomed Pharmazeutische und Medizintechnische Gesellschaft mbH, D-85609 Dornach (DE)
(72) Erfinder: Meinert, Hasso, Prof. Dr., W-7900 Ulm (DE)
(74) Vertreter: Nöth, Heinz, Dipl.-Phys.

(56) Entgegenhaltungen:
- EP-A- 0 089 815
- WO-A-87/01271
- AMERICAN JOURNAL OF OPHTHALMOLOGY, Band 103, Nr. 1, Januar 1987, Seiten 38-43; S. CHANG: "Low Viscosity Liquid Fluorochemicals in Vitreous Surgery"
- AMERICAN JOURNAL OF OPHTHALMOLOGY, Band 106, Nr. 6, Dezember 1988, Seiten 668-674, Chicago, US; S. CHANG et al.: "Intraoperative Perfluorocarbon Liquids in the Management of Proliferative Vitreoretinopathy"
- KLIN. MBL AUGENHEILK., Band 192, 1988, Seiten 277- 283; H. LAQUA et al.: "Entwicklung und gegenwärtiger Stand der Silikonölchirurgie"

## Beschreibung

Die Erfindung betrifft ein Verdünnungsmittel für zur Netzhautentfaltung eingesetzte Perfluorcarbone (Entfaltungs-PFCL).

Der Einsatz von flüssigen Perfluorcarbonen (Entfaltungs-PFCL) als Behandlungsflüssigkeit ist beispielsweise aus der US-PS 4 490 391 bekannt. Der Einsatz von PFCL bei der Behandlung von Netzhautablösung und von großen Rissen in der Netzhaut hat sich aufgrund der hohen chemischen Stabilität und der Dichten im Bereich von 1,5 - 1,8 g/cm³ dieser Stoffe empfohlen. Bei der Netzhautentfaltung werden nach Entfernen des Glaskörpers die flüssigen PFCLs in das Auge eingebracht und, während der Patient auf dem Rücken liegt, drücken diese Flüssigkeiten aufgrund ihrer Dichte die Netzhaut bzw. die mit Rissen behaftete Netzhaut wieder an das Aderhautgewebe (Choroidgewebe) des Auges an. Nach einer Verweilzeit von mehreren Stunden wird das PFCL wieder abgesaugt und durch ein anderes Medium, beispielsweise Methylsilikonöl, ersetzt. In diesem Zusammenhang kann auf die im folgenden noch aufgelistete Literatur hingewiesen werden:
H. Lacua, K. Lucke, M.H. Foerster "Entwicklung und gegenwärtiger Stand der Silikonölchirurgie" in Klin. Mbl. Augenheilk. 192, 1988, S. 277 - 283; A. Kampik "Prophylaxe und Behandlung der proliferativen Vitreoretinopathien" in Z. prakt. Augenheilkd. 7 S. 323 - 326 (1986); Stanley Chang, Emin Ozmert, Neal J. Zimmermann "Intraoperative Perfluorcarbon liquids in the Management of Proliferative Vitreoretinopathy in "American Journal of Ophthalmology 106 (Dec. 1988) S. 668 - 674; Stanley Chang, "Low Viscosity Liquid Fluorochemicals in Vitreous Surgery" in American Journal of Ophthalmology 103 (January 1987) S. 38-43; Anselm Kampik "Klinik und Pathogenese der Windenblütenablatio" in Z. prakt. Augenheilkd. 4 (1983) S. 371 - 378; Klaus Lucke "Vitreorotinale Chirurgie bei komplizierten Netzhautablösungen" in Z. prakt. Augenheilkd. 9 (1988) S. 137 - 147).

Es hat sich nun herausgestellt, daß die Entfaltungs- PFCL nicht rückstandsfrei abgesaugt werden können. Es verbleiben noch Reste in Tröpfchenform im Auge. Diese können über längere Zeit hin zu Schädigungen des Glaskörpers und der Linse führen. Die rückstandsfreie Beseitigung der Entfaltungs-PFCL ist nicht so ohne weiteres möglich, da Perfluorcarbone mit herkömmlichen Lösungsmitteln nicht beseitigt werden können.

Aufgabe der Erfindung ist es daher, ein Mittel zu schaffen, mit dem Perfluorcarbone, welche als Entfaltungsflüssigkeit zur Netzhautentfaltung eingesetzt worden sind, rückstandsfrei aus dem Auge entfernt werden können.

Diese Aufgabe wird erfindungsgemäß durch ein Verdünnungsmittel für zur Netzhautentfaltung eingesetzte Perfluorcarbone (Entfaltungs-PFCLs) gelöst, das als bei Zimmertemperatur leichter flüchtiges als Entfaltungs-PFCLs und niederviskoses Perfluorcarbon (Verdünnungs-PFCL) mit einem Siedepunkt im Bereich von Körpertemperatur (ca. 38° C) bis 100° C ausgebildet ist.

In vorteilhafter Weise kann dieses Verdünnungsmittel als Lösungsmittel für im Auge verbliebene Reste an Entfaltungs-PFCLs eingesetzt werden. Perfluorcarbone lassen sich in Perfluorcarbonsystemen lösen. Bei der Erfindung ist zur Beseitigung der Restentfaltungs-PFCLs aus dem Auge das Lösungs- bzw. Verdünnungsmittel jedoch in der Weise ausgebildet, daß es beispielsweise mittels einer Kanüle und Injektionsspritze in das Auge zur Entfernung der Restentfaltungsflüssigkeit eingebracht werden kann, und ferner einen relativ niedrigen Siedepunkt aufweist, der oberhalb der Körpertemperatur zwischen 38° C und 100° C liegt. Dieses Verdünnungsmittel wirkt als Lösungsmittel für die Restanteile der zur Netzhautentfaltung eingesetzten Entfaltungsflüssigkeit (Entfaltungs-PFCLs). Insbesondere dann, wenn zur Netzhautentfaltung relativ schwerflüchtige und viskose PFCLs mit niedrigem Dampfdruck eingesetzt werden, erweist sich die Entfernung von Restanteilen derartiger PFCLs mit Hilfe des erfindungsgemäßen Verdünnungsmittels als vorteilhaft.

Das Entfernen der Restanteile an Entfaltungs-PFCLs kann so erfolgen, daß mittels einer Kanüle und Injektionsspritze ca. 1 bis 3 cm³ Verdünnungs-PFCLs eingegeben werden. Es lösen sich dann in einer Verweilzeit von nur wenigen Minuten Restanteile des Entfaltungs-PFCLs im Verdünnungsmittel. Das Gemisch kann dann über die Kanüle auf gleichem Wege wieder abgesaugt werden. Ferner wird durch das erfindungsgemäße Verdünnungsmittel erreicht, daß das Gemisch aus dem Verdünnungsmittel und den restlichen Entfaltungs-PFCLs aufgrund des niedrigen Siedepunktes bzw. hohen Dampfdruckes verdampfen und sich rasch verflüchtigen. Die im Verdünnnungsmittel vorher aufgenommenen schwerflüchtigen Entfaltungs-PFCLs-Anteile werden aufgrund des Verdünnungsgesetztes mit in die Gasphase übergeführt.

Sollten in der Gasphase im Auge noch geringfügige Mengen an PFC-Molekülen verbleiben, werden diese resorbiert und verlassen so das Auge.

Es ist auch möglich, daß nach dem Abziehen des Verdünnungsmittels zunächst noch einige Minuten gewartet wird, um das Verdampfen der Restanteile des Verdünnungsmittels mit den gelösten Entfaltungs-PFCLs bei der Temperatur des Auges herbeizuführen und anschließend über die bis dahin im Auge verbliebene Kanüle noch eine Spülung mit einem inerten Gas, beispielsweise Stickstoff oder Edelgas, oder auch mit Luft vorzunehmen. Durch diesen zusätzlichen Vorgang lassen sich restliche Anteile an PFC-Molekülen ebenfalls rasch aus dem Auge entfernen.

Insbesondere kann das Verdünnungsmittel auch als Lösungsmittel für im Auge noch verbliebene Restanteile an Entfaltungs-PFCLs, wie sie in der deutschen Patentanmeldung P 41 00 059.5 beschrieben sind, verwendet werden.

Beispiele für leichtflüchtige, niederviskose PFCL, welche als Verdünnungsmittel zum Einsatz kommen können, sind Perfluoralkane mit wenigstens 6 Kohlenstoffatomen im Molekül, insbesondere Perfluorhexan (C₆ F₁₄) mit einem Siedepunkt von ca. 60° C oder Perfluorheptan (C₇ F₁₆) mit einem Siedepunkt von etwa 82° C. Ferner eignen sich Perfluortrialkylamine, wie beispielsweise Perfluor-triethylamin (N(C₂F₅)₃) mit einem Siedepunkt von ca. 70° C oder Perfluordialkylether, wie beispielsweise Perfluordipropylether (C₃F₇-O-C₃F₇) mit einem Siedepunkt von ca. 60° C.

Das Verdünnungsmittel kann auch in der Weise zur Verfügung gestellt werden, daß es in einem Behandlungssystem als Verdünnungs-PFCL mit einem Entfaltungs-PFCL kombiniert ist. Hierzu befinden sich das Entfaltungs-PFCL und das Verdünnungs-PFCL in getrennten Behältern und werden für die durchzuführende Behandlung bei der Netzhautentfaltung so in Bereitschaft gehalten.

## Patentansprüche

1. Verdünnungsmittel für zur Netzhautentfaltung eingesetzte Perfluorcarbone (Entfaltungs-PFCL), gekennzeichnet durch ein bei Zimmertemperatur leichter flüchtiges und niederviskoses Perfluorcarbon (Verdünnungs-PFCL) mit einem Siedepunkt im Bereich von Körpertemperatur (ca. 38° C) bis 100° C.

2. Verdünnungsmittel nach Anspruch 1, gekennzeichnet als Lösungsmittel für im Auge verbliebene Restanteile an Entfaltungs-PFCL.

3. Verdünnungsmittel nach Anspruch 2, gekennzeichnet durch eine Dosiermenge von 1 bis 3 cm³.

4. Verdünnungsmittel nach einem der Ansprüche 1 bis 3, gekennzeichnet durch ein Perfluoralkan mit wenigstens 6 Kohlenstoffatomen im Molekül.

5. Verdünnungsmittel nach Anspruch 4, dadurch gekennzeichnet, daß das Perfluoralkan Perfluorhexan oder Perfluorheptan ist.

6. Verdünnungsmittel nach einem der Ansprüche 1 bis 3, gekennzeichnet durch ein Perfluor-trialkylamin.

7. Verdünnungsmittel nach Anspruch 6, dadurch gekennzeichnet, daß das Perfluor-trialkylamin ein Perfluor-triethylamin ist.

8. Verdünnungsmittel nach einem der Ansprüche 1 bis 3, gekennzeichnet durch einen Perfluordialkylether.

9. Verdünnungsmittel nach Anspruch 8, dadurch gekennzeichnet, daß der Perfluordialkylether ein Perfluordipropylether ist.

10. Verdünnungsmittel nach einem der Ansprüche 1 bis 9, dadurch gekennzeichnet, daß es als Verdünnungs-PFCL ein in einem Behälter gesondert aufbewahrter Bestandteil eines Behandlungssystems ist, das noch das ebenfalls in einem Bereitschaftsbehälter aufbewahrte Entfaltungs-PFCL aufweist.

11. Verdünnungsmittel nach Anspruch 10, dadurch gekennzeichnet, daß das Verdünnungs-PFCL in einer Dosismenge von 1 bis 3 cm³ in Bereitschaft gehalten ist.

## Claims

1. A diluent for perfluorocarbons (deployment PFCL) used for retinal deployment, characterized by a perfluorocarbon (diluent PFCL) that is more easily volatile and of low viscosity at room temperature and has a boiling point in the range of body temperature (approx. 38°C) to 100°C.

2. A diluent according to claim 1, characterized as a solvent for residual amounts of deployment PFCL remaining in the eye.

3. A diluent according to claim 2, characterized by a dosage of 1 to 3 cm³.

4. A diluent according to any one of claims 1 to 3, characterized by a perfluoroalkane with at least 6 carbon atoms in the molecule.

5. A diluent according to claim 4, characterized in that the perfluoroalkane is perfluorohexane or perfluoroheptane.

6. A diluent according to any one of claims 1 to 3, characterized by a perfluorotrialkylamine.

7. A diluent according to claim 6, characterized in that the perfluorotrialkylamine is a perfluorotriethylamine.

8. A diluent according to any one of claims 1 to 3, characterized by a perfluorodialkylether.

9. A diluent according to claim 8, characterized in that the perfluorodialkylether is a perfluorodipropylether.

10. A diluent according to any one of claims 1 to 9, characterized in that, as a diluent PFCL it is a component, separately stored in a container, of a treatment system that also includes the deployment PFCL which is also stored in a standby container.

11. A diluent according to claim 10, characterized in that the diluent PFCL is held in readiness in a dosage of 1 to 3 cm³.

## Revendications

1. Diluant pour des perfluorocarbures à utiliser dans le déploiement de la rétine (PFCL de déploiement) , caractérisé par un perfluorocarbure (PFCL de dilution) facilement volatil et faiblement visqueux à la température ambiante, présentant un point d'ébullition dans l'intervalle depuis la température corporelle (environ 38°C) jusqu'à 100°C.

2. Diluant selon la revendication 1, caractérisé en ce qu'il s'agit d'un solvant pour les parties résiduelles de PFCL de déploiement restant dans l'oeil.

3. Diluant selon la revendication 2, caractérisé par une quantité de dosage de 1 à 3 cm³.

4. Diluant selon l'une des revendications 1 à 3, caractérisé par un perfluoroalcane comportant au moins 6 atomes de carbone dans la molécule.

5. Diluant selon la revendication 4, caractérisé en ce que le perfluoroalcane est le perfluorohexane ou le perfluoroheptane.

6. Diluant selon l'une des revendications 1 à 3, caractérisé par une perfluorotrialkylamine.

7. Diluant selon la revendication 6, caractérisé en ce que la perfluorotrialkylamine est une perfluorotriéthylamine.

8. Diluant selon l'une des revendications 1 à 3, caractérisé par un perfluorodialkyléther.

9. Diluant selon la revendication 8, caractérisé en ce que le perfluorodialkyléther est un perfluorodipropyléther.

10. Diluant selon l'une des revendications 1 à 9, caractérisé en ce qu'en tant que PFCL de dilution, il comprend un composant conservé dans un récipient approprié et faisant partie d'un système de traitement qui comporte en outre le PFCL de déploiement stocké également dans un récipient disponible.

11. Diluant selon la revendication 10, caractérisé en ce que le PFCL de dilution est conservé disponible dans une quantité de dosage de 1 à 3 cm³.
